# EUROPÄISCHE PATENTANMELDUNG

(11) **EP 1 527 808 A1**
(43) Veröffentlichungstag der Anmeldung: **04.05.2005**
(21) Anmeldenummer: 04025472.4
(22) Anmeldetag: 27.10.2004
(51) Int. Cl.: B01D 51/10

(54) **Vorrichtung und Verfahren zur Konditionierung eines Gasgemisches**

(30) Priorität: 27.10.2003 AT 16972003
(71) Anmelder: GE Jenbacher GmbH & Co. OHG, 6200 Jenbach (AT)
(72) Erfinder: Klane, Bernd, 6262 Bruck am Ziller (AT); Schiliro, Michele, 6200 Jenbach (AT)
(74) Vertreter: Torggler, Paul N.

(57) **Zusammenfassung**

Vorrichtung zur Konditionierung eines Gasgemisches, insbesondere von Biogas für Verbrennungsmaschinen, dadurch gekennzeichnet, dass die Vorrichtung eine Wascheinrichtung (1) mit kaltem oder gekühltem Wasser, einen dieser vorgeschalteten Verdichter (9) und eine dieser nachgeschaltene Entspannungseinrichtung (12) aufweist.

## Beschreibung

Die vorliegende Erfindung betrifft eine Vorrichtung sowie ein Verfahren zur Konditionierung eines Gasgemisches, insbesondere von Biogas für Verbrennungsmaschinen.

In Gärbehältern wird aus organischen Substraten, wie Klärschlamm, Gülle, Lebensmittelabfällen und Pflanzenschnitt energiereiches Biogas gewonnen. Dieses Biogas kann in Maschinen, wie Gasmotoren und Turbinen oder zukünftig in elektrochemischen Energiewandlern, wie zB Brennstoffzellen, in Wärme und elektrische Energie umgewandelt werden. Das Biogas aus dem Fermenter enthält neben den gewünschten Komponenten wie Methan oder Wasserstoff auch unerwünschte Gaskomponenten, die den Betrieb von Maschinen erschweren. Dies sind beispielsweise Schwefelwasserstoff (H₂S) und/oder Ammoniak (NH₃). Auch Wasserdampf ist hinderlich, wenn er in einer Menge vorliegt, dass beim Eintritt in die Maschine Kondensation auftritt. Das Biogas verlässt den Fermenter üblicherweise mit ca. 37°C, 100% relative Feuchte und ca. 5 mbar Überdruck. Für den wirtschaftlichen Maschinenbetrieb ist ein Biogas vorteilhaft, das arm an den genannten Stoffen ist, eine mäßige relative Feuchte aufweist, nicht wärmer als ca. 40°C und am Maschineneintritt mit Überdruck (z.B. 50 mbar bis 200 mbar) vorliegt. Um das Biogas in diesen Zustand zu bringen, sind oft mehrere Verfahren mit verschiedenen Anlagenkomponenten im Einsatz. Der Stand der Technik offenbart beispielsweise Anlagen mit biologischen oder chemischen Entschwefelungsreaktoren mit nachgeschaltetem Verdichter, einer Gaskühlstrecke zur Kondensation von Wasser und einer Vorwärmstrecke zur Senkung der relativen Feuchte. Die Gesamtheit solcher Anlagen stellt einen beträchtlichen Investitions- und Betriebsmittelaufwand dar, der die Wirtschaftlichkeit der Biogasanlage belastet. Auch die Abstimmung der einzelnen Anlagenkomponenten aufeinander gelingt in der Praxis oft nicht zufriedenstellend, wenn nicht ein unverhältnismäßig großer Aufwand zur Prozessregelung betrieben wird.

Aufgabe der vorliegenden Erfindung ist es daher, eine Vorrichtung zur Konditionierung von Gasgemischen vorzuschlagen, die sich durch eine geringe Anzahl und Größe der Anlagenkomponenten auszeichnet und die die Bereitstellung eines optimalen Gasgemisches, insbesondere für Verbrennungsmaschinen, sicherstellt.

Erfindungsgemäß wird dies dadurch erreicht, dass die Vorrichtung eine Wascheinrichtung mit kaltem oder gekühltem Wasser, einen dieser vorgeschalteten Verdichter und eine dieser nachgeschaltene Entspannungseinrichtung aufweist.

Es ist bereits bekannt, dass sich durch erhöhten Druck und Temperatur des Gasgemisches eine bessere Absorption von Ammoniak (NH₃) in einer nachgeschalteten Wascheinrichtung erzielt werden kann. Durch die erfindungsgemäße Vorrichtung zur Konditionierung eines Gasgemisches mit den Komponenten Verdichten/Kaltwaschen/Entspannen wird zuerst das Gasgemisch durch den Verdichter auf einen unüblich hohen Druck und hohe Temperatur verdichtet, wodurch eine niedrigere Wasserbeladung erreicht wird. Die Kondensation bzw. Lösung von Spurenstoffen sowie die Abscheidung von Wasserdampf in der Wascheinrichtung wird dadurch erhöht. Bei der hohen Temperatur ist die Abgabe von Energie aus dem Gasgemisch leicht möglich. Durch diese effektiven Prozessabläufe können die Apparate kleiner und daher kostengünstiger realisiert werden. Günstigerweise ist vorgesehen, dass das kalte oder gekühlte Wasser kälter als das Gasgemisch ist. Dadurch wird in der Wascheinrichtung das Gas(Luft)-Dampf-Gemisch durch das kalte oder gekühlte Wasser unter seinem Taupunkt (Sättigungstemperatur) abgekühlt, so dass ein Teil des Dampfes zu Flüssigkeit wird. Diese Flüssigkeit kann als Nebel im Gemisch austauen (ausregnen und mit dem Waschwasser vermischen oder an Kühlflächen niederschlagen). Von den Kühlflächen tropft oder rieselt Flüssigkeit ab. Das feuchte Gas vermindert also seine absolute Feuchte, indem Flüssigkeit austaut und dadurch wird die Beladung des Gasgemisches mit Wasser (Massenbeladung in g H₂O pro kg Gasgemisch) in der Wascheinrichtung gesenkt. Bei der folgenden Teilentspannung in der Entspannungseinrichtung wird die volumetrische Wasserbeladung (in g H₂O pro m³ Biogas) effektiv gesenkt. Diese ist, bei gegebener Temperatur, maßgeblich für die relative Feuchte.

Es kann auch vorgesehen sein, dass vor dem Verdichter ein Tropfenabscheider angeordnet ist. Dadurch werden aus dem Fermenter mitgerissene Flüssigkeitspartikel entfernt. Dabei ist der Abscheidegrad wesentlich von der Größe und Dichte der abzuscheidenden Tropfen und der Zähigkeit des Gasgemisches abhängig. Um eine vorteilhafte Abscheidung zu erreichen, können die Tropfenabscheider zusätzlich mit Aerosolabscheidern ausgerüstet werden.

Besonders vorteilhaft ist vorgesehen, dass nach dem Verdichter ein Wärmetauscher angeordnet ist, der das in die Wascheinrichtung eintretende Gasgemisch kühlt. Das vorverdichtete Gas gibt damit Wärme an das aus der Wascheinrichtung kommende entspannte Gas ab, und dadurch kann die relative Feuchte des entspannten Gases gesenkt werden, wobei gleichzeitig das in die Wascheinrichtung eintretende Gas gekühlt wird. Dies kann bevorzugt mit einem einzigen Wärmetauscher realisiert werden, es sind aber genauso zwei separat angeordnete Wärmetauscher denkbar. Genauso ist es denkbar, die Erwärmung des entspannten Gases mit Hilfe einer anderen Wärmequelle, beispielsweise mit Motorkühlwasser, durchzuführen.

Vorteilhaft ist vorgesehen, dass die Wascheinrichtung einen Wäscher mit kaltem oder gekühltem Wasser aufweist. Durch diese Anordnung kann die Wasserbeladung des Gasgemisches gesenkt werden, da durch das kalte oder gekühlte Wasser das Gasgemisch derart abgekühlt wird, dass ein Teil des Dampfes zu Flüssigkeit wird und mit dem Waschflüssigkeitsgemisch abfließen kann. Eine bevorzugte Ausführungsform der Erfindung sieht vor, dass die Wascheinrichtung einen im Wesentlichen geschlossenen Wasserkreislauf mit einer Förderpumpe aufweist. Dadurch wird das Gasgemisch kontinuierlich einem Wasserkreislauf ausgesetzt, wodurch dem Gasgemisch im Wäscher laufend Feuchtigkeit entzogen wird. Vorteilhaft ist vorgesehen, dass im Wasserkreislauf, vorzugsweise außerhalb des Wäschers, eine Kühlstufe angeordnet ist. Dadurch wird einerseits verhindert, dass sich das Wasser durch das heiße Gasgemisch zu stark erwärmt und andererseits wird eine Temperatur unter der Sättigungstemperatur zum Austauen von Flüssigkeit aus dem Gasgemisch ermöglicht. Dadurch, dass der Wäscher eine Beregnungseinrichtung umfasst, kann das Gasgemisch beispielsweise mit einer wässrigen Waschlösung ausgewaschen werden. Der Vorteil der Waschlösung gegenüber einem reinen Wasserbad liegt darin, dass unerwünschte Gaskomponenten, wie z.B. Schwefelwasserstoff (H₂S), besser gelöst werden können, sodass insgesamt weniger Flüssigkeit verbraucht wird und der energetische Aufwand für die Umwälzung sinkt. Vorteilhaft ist vorgesehen, dass das Waschwasser mit chemischen Reagentien versehen wird, die die Aufnahme der Spurenkomponenten erhöhen (z.B. Eisenchloride, Säuren, Basen). Vorteilhaft ist eine Frischwasserzufuhr vorgesehen, über die Frischwasser dem Wäscher zuführbar ist. Das in den Wasserkreislauf eingespeiste Frischwasser wird mit dem mit Spurenstoffen angereicherten Wasser vermischt, was zu einer verbesserten Absorptionskapazität hinsichtlich der unerwünschten Gaskomponenten führt. Günstigerweise ist ein Ablauf vorgesehen, über den mit Schadstoffen angereichertes Wasser aus dem Wäscher abführbar ist, damit die Spurenstoffe aus dem Wasserkreislauf ausgeschieden werden können.

Die Gaswäsche, bei der die absolute Wasserbeladung und die Gastemperatur effektiv gesenkt werden, kann durch mehrere Varianten ausgeführt werden. Bei einer Variante wird das Waschwasser zirkuliert und gibt dabei ständig Wärme (sensible Wärme und Kondensationswärme) auf hohem Temperaturniveau an die Umgebung ab, wobei aus dem Gasgemisch kondensierendes Wasser abgeführt werden muss. Die Abgabe von Wärme an die Umgebung kann beispielsweise über ein Rohrregister realisiert werden. Gemäß einem weiteren Ausführungsbeispiel kann ständig kaltes Wasser bzw. eine kalte Waschlösung dem Wasserkreislauf zugeführt werden. Bei einer weiteren Variante der Gaswäsche wird eine Teilmenge des zirkulierenden Waschwassers kontinuierlich ausgetauscht. Das abgeführte Waschwasser nimmt aus dem Gasgemisch aufgenommene Wärme und wasserlösliche Spurenstoffe wie Schwefelwasserstoff (H₂S) und Ammoniak (NH₃) mit. Es kann auch vorgesehen sein, dass eine Kühlmaschine die zirkulierende Waschlösung kühlt. Bei allen Varianten der Gaswäsche kann ein biologischer Entschwefelungsprozess auftreten. Da der Schwefelgehalt von Gasen die Lebensdauer von Leitungen und Verbrauchseinrichtungen beeinträchtigen kann, ist ein solcher Entschwefelungsprozess vorteilhaft. Selbstverständlich können optional auch chemische Absorptionstechniken zum Zuge kommen.

Gemäß einem weiteren Ausführungsbeispiel der Erfindung kann vorgesehen sein, dass die Entspannungseinrichtung mindestens eine Entspannungsmaschine wie eine Turbine aufweist, die über eine Welle mit einem Laufrad des Verdichters verbindbar ist. Dadurch wird Antriebsleistung für den Verdichter gespart und die Temperatur des Gasgemisches ist vorteilhaft niedriger. Die Entspannungseinrichtung kann auch mindestens eine Drossel (beispielsweise ein Joule-Thomson Ventil) aufweisen, die das Gasgemisch vorteilhaft entspannt. Dabei wird das Gasgemisch unterhalb der Inversionstemperatur durch isenthalpe Expansion abgekühlt.

Besonders vorteilhaft ist vorgesehen, dass das entspannte Gasgemisch nach der Konditionierung einer angeschlossenen Verbrennungsmaschine, vorzugsweise einem Biogasmotor, als konditioniertes Gas zuführbar ist. Dieses Gas weist hinsichtlich jeder Zustandsgröße optimale Werte auf, die einen vorteilhaften Betrieb eines Gasmotors hinsichtlich optimaler Verbrennungseigenschaften ermöglichen. Die Anzahl und Größe der Anlagenkomponenten und der einzusetzenden Hilfsenergie und Hilfsstoffe ist vergleichsweise gering. Auch die zu steuernden, zu regelnden und zu überwachenden Betriebsparameter sind einfache Größen und deren Anzahl ist ebenfalls gering.

Das erfindungsgemäße Verfahren ist dadurch gekennzeichnet, dass das Gasgemisch zuerst verdichtet, danach mit kaltem und/oder mit gekühltem Wasser in Kontakt gebracht, vorzugsweise gewaschen, und danach wieder entspannt wird.

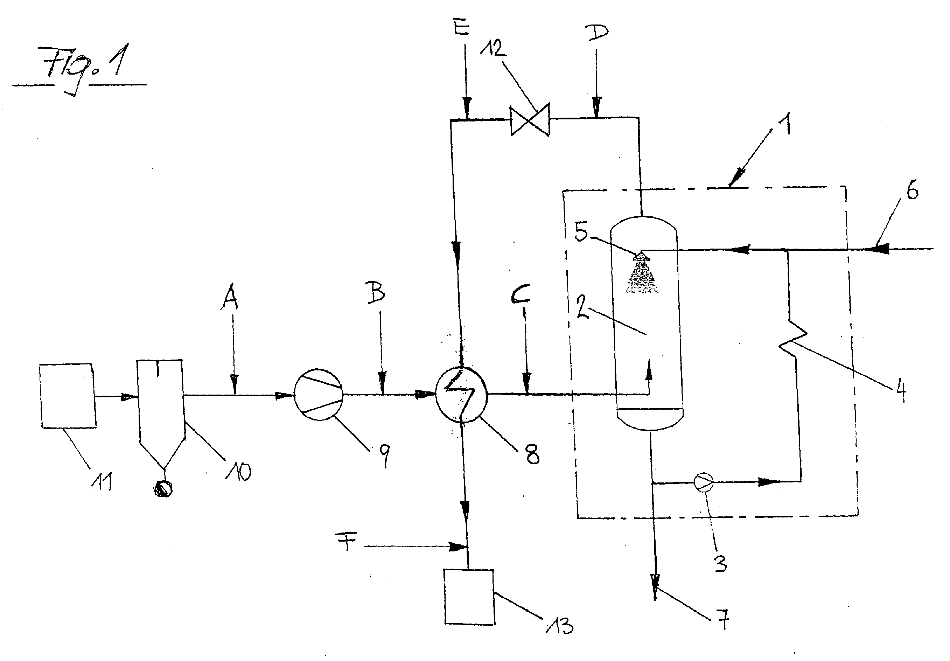

Weitere Einzelheiten und Vorteile der vorliegenden Erfindung werden anhand der Figurenbeschreibung unter Bezugnahme auf die Zeichnung im Folgenden näher erläutert. Darin zeigt die Fig. 1 eine schematische und beispielhafte Darstellung der erfindungsgemäßen Anlage zur Konditionierung eines Gasgemisches.

Das in Fig. 1 gezeigte Ausführungsbeispiel einer Vorrichtung zur Konditionierung eines Gasgemisches weist erfindungsgemäß eine Wascheinrichtung 1 mit kaltem oder gekühltem Wasser (innerhalb der strichpunktierten Linie gekennzeichnet), einer dieser vorgeschalteten Verdichter 9 und eine dieser nachgeschaltene Entspannungseinrichtung 12 auf. auf. Ein von einer Biogasquelle 11 kommendes Gasgemisch durchströmt einen Tropfenabscheider 10, wodurch Flüssigkeitspartikel aus dem Luftstrom entfernt werden. Der nachgeschaltete Verdichter 9 erhöht Druck des Gasgemisches, wodurch die Kondensation und Abscheidung von Wasserdampf in der nachgeschalteten Wascheinrichtung 1 erhöht wird. Durch die hohe Temperatur des verdichteten Gasgemisches ist die Abgabe von Energie aus dem Gasgemisch an die Umgebung oder an einen Waschwasserstrom leicht möglich. Die Wascheinrichtung 1 an sich weist einen Wäscher 2 mit kaltem oder gekühltem Wasser auf, der einen im Wesentlichen geschlossenen Behälter mit einer Beregnungseinrichtung 5 vorsieht. Diese kann weiters eine Füllkörperschüttung oder eine Packung aufweisen, die die Austauschfläche zwischen Gas und Waschlösung vergrößert. Die Wascheinrichtung 1 weist zudem einen im Wesentlichen geschlossenen Wasserkreislauf mit einer Förderpumpe 3 und einer Kühlstufe 4 auf, die den Wäscher 2 mit gekühltem Wasser versorgt. Da vorgesehen ist, dass das kalte oder gekühlte Wasser kälter als das Gasgemisch ist, kann die Kühlstufe 4 beispielsweise als einfaches Rohrregister ausgeführt sein, welches ständig Wärme aus dem Wasser an die Umgebung abgeben kann. Eine Frischwasserzufuhr 6 führt dem Wasserkreislauf Frischwasser zur besseren Absorption von Spurenstoffen zu. Vorteilhaft ist ein Ablauf 7 vorgesehen, über den mit ausgewaschenen Schadstoffen angereichertes Wasser aus dem Wäscher 2 abführbar ist. Ein in den Wäscher 2 eintretendes Gasgemisch wird durch die Zirkulation mit gekühltem Wasser hinsichtlich seiner absoluten Feuchte reduziert, da das Gasgemisch durch das kalte oder gekühlte Wasser unter der Sättigungstemperatur abgekühlt wird und dadurch Flüssigkeit austaut. Zudem gibt das Gasgemisch neben Wasser auch Wärme und gegebenenfalls auch Spurenstoffe an die Waschlösung ab. Das aus dem Wäscher 2 austretende Gasgemisch wird durch die Entspannungseinrichtung 12 entspannt, wobei Druck und Temperatur gesenkt werden, und damit wird eine niedrigere relative Feuchte des Gasgemisches erzielt. Es kann eine Entspannungskraftmaschine oder eine Drossel (beispielsweise ein Joule-Thomson-Ventil) eingesetzt werden. Vorteilhaft ist vorgesehen, dass die Entspannungseinrichtung mindestens eine Entspannungsmaschine wie eine Turbine aufweist. Besonders vorteilhaft ist vorgesehen, dass diese über eine Welle mit dem Laufrad des Verdichters 9 gekoppelt wird. Dadurch wird Antriebsleistung für den Verdichter 9 gespart und die Temperatur des Gasgemisches wird vorteilhaft gesenkt. Durch die Teilentspannung in der Entspannungseinrichtung 12 wird die volumetrische Wasserbeladung effektiv gesenkt. Diese ist, bei gegebener Temperatur, maßgeblich für die relative Feuchte. Ein nachgeschalteter Wärmetauscher 8 gibt die Wärme des verdichteten Gasgemisches an das entspannte Gasgemisch ab. Dadurch kann die relative Feuchte des entspannten Gases gesenkt werden und das entspannte Gasgemisch kann nach der Konditionierung einer nachgeschalteten Verbrennungsmaschine 13, vorzugsweise einem Biogasmotor, für eine optimale Verbrennung zugeführt werden. Dieses konditionierte Gas weist hinsichtlich jeder Zustandsgröße einen günstigen Gaszustand auf, der einen vorteilhaften Betrieb der Verbrennungsmaschine ermöglicht.

Nachstehende Tabelle zeigt beispielhafte Werte an verschiedenen Messpunkten A, B, C, D, E, F an der in Fig. 1 dargestellten Vorrichtung.

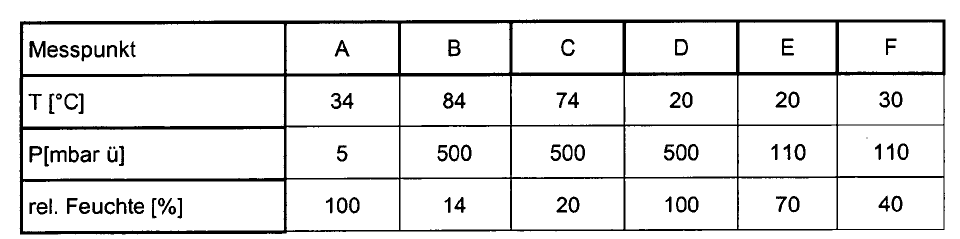

Aus Gärbehältern tritt das Biogas üblicherweise in einem Zustand aus, der folgendermaßen beschrieben werden kann (Messpunkt A):
- das Gasgemisch liegt mit einer Temperatur zwischen 20° und 50°, vorzugsweise zwischen 30° und 40°, vor.
- das Gasgemisch weist einen Überdruck zwischen 0 mbar und 20 mbar, vorzugsweise zwischen 0 mbar und 10 mbar, auf.
- das Gasgemisch weist eine relative Feuchte zwischen 90% und 100% auf.

Vorteilhaft ist vorgesehen, dass:
Bei Messpunkt B:
   - das Gasgemisch vor Eintritt in den Wärmetauscher eine Temperatur zwischen 75° und 150°, vorzugsweise zwischen 80° und 120°, aufweist;
   - das Gasgemisch vor Eintritt in den Wärmetauscher einen Überdruck zwischen 200 mbar und 2000 mbar, vorzugsweise zwischen 400 mbar und 1000 mbar, aufweist;
   - das Gasgemisch vor Eintritt in den Wärmetauscher eine relative Feuchte zwischen 5% und 25%, vorzugsweise zwischen 10% und 20%, aufweist.
Bei Messpunkt C:
   - das Gasgemisch vor Eintritt in die Wascheinrichtung eine Temperatur zwischen 50° und 140°, vorzugsweise zwischen 60° und 100°, aufweist;
   - das Gasgemisch vor Eintritt in die Wascheinrichtung einen Überdruck zwischen 100 mbar und 2000 mbar, vorzugsweise zwischen 400 mbar und 1000 mbar, aufweist;
   - das Gasgemisch vor Eintritt in die Wascheinrichtung eine relative Feuchte zwischen 10% und 30%, vorzugsweise zwischen 15% und 25%, aufweist.
Bei Messpunkt D:
   - das Gasgemisch nach Austritt aus der Wascheinrichtung eine Temperatur zwischen 5° und 45°, vorzugsweise zwischen 15° und 25°, aufweist;
   - das Gasgemisch nach Austritt aus der Wascheinrichtung einen Überdruck zwischen 100 mbar und 2000 mbar, vorzugsweise zwischen 400 mbar und 1000 mbar, aufweist;
   - das Gasgemisch nach Austritt aus der Wascheinrichtung eine relative Feuchte zwischen 90% und 100%, üblicherweise zwischen 95% und 100%, aufweist.
Bei Messpunkt E:
   - das Gasgemisch nach dem Austritt aus der Entspannungseinrichtung eine Temperatur zwischen 10° und 45°, vorzugsweise zwischen 15° und 25°, aufweist;
   - das Gasgemisch nach dem Austritt aus der Entspannungseinrichtung einen Überdruck zwischen 20 mbar und 300 mbar, vorzugsweise zwischen 80 mbar und 150 mbar, aufweist;
   - das Gasgemisch nach dem Austritt aus der Entspannungseinrichtung eine relative Feuchte zwischen 30% und 90%, vorzugsweise zwischen 40% und 80%, aufweist.
Bei Messpunkt F:
   - das Gasgemisch vor Eintritt in die Verbrennungsmaschine eine Temperatur zwischen 15° und 60°, vorzugsweise zwischen 25° und 40°, aufweist;
   - das Biogas vor Eintritt in die Verbrennungsmaschine einen Überdruck zwischen 20 mbar und 300 mbar, vorzugsweise zwischen 80 mbar und 150 mbar, aufweist;
   - das Biogas in die Verbrennungsmaschine eine relative Feuchte zwischen 30% und 90%, vorzugsweise zwischen 50% und 80%, aufweist.

Es versteht sich von selbst, dass die vorliegende Erfindung nicht auf das in der Figur dargestellte Ausführungsbeispiel beschränkt ist, noch durch dieses eingeschränkt werden soll. Auch die in der Tabelle angeführten Werte sind als grobe Richtlinien, aber keinesfalls als beschränkende Angaben zu betrachten. Die Erfindung bezieht sich nicht nur auf die oben beschriebene Vorrichtung, sondern auch auf die erläuterten Verfahrensschritte.

## Patentansprüche

1. Vorrichtung zur Konditionierung eines Gasgemisches, insbesondere von Biogas für Verbrennungsmaschinen, **dadurch gekennzeichnet, dass** die Vorrichtung eine Wascheinrichtung (1) mit kaltem oder gekühltem Wasser, einen dieser vorgeschalteten Verdichter (9) und eine dieser nachgeschaltene Entspannungseinrichtung (12) aufweist.

2. Vorrichtung nach Anspruch 1, **dadurch gekennzeichnet, dass** das kalte oder gekühlte Wasser kälter als das Gasgemisch ist.

3. Vorrichtung nach Anspruch 1 oder 2, **dadurch gekennzeichnet, dass** das Gasgemisch vor Eintritt in den Verdichter (9) eine Temperatur zwischen 20° und 50°, vorzugsweise zwischen 30° und 40°, aufweist.

4. Vorrichtung nach einem der Ansprüche 1 bis 3, **dadurch gekennzeichnet, dass** nach dem Verdichter ein Wärmetauscher (8) angeordnet ist, der das in die Wascheinrichtung (1) eintretende Gasgemisch kühlt.

5. Vorrichtung nach einem der Ansprüche 1 bis 4, **dadurch gekennzeichnet, dass** ein Wärmetauscher (8) vorgesehen ist, der das aus der Wascheinrichtung (1) kommende Gasgemisch erwärmt.

6. Vorrichtung nach einem der Ansprüche 1 bis 5, **dadurch gekennzeichnet, dass** die Wascheinrichtung (1) einen im Wesentlichen geschlossenen Wasserkreislauf mit einer Förderpumpe (3) aufweist.

7. Vorrichtung nach einem der Ansprüche 1 bis 6, **dadurch gekennzeichnet, dass** die Wascheinrichtung (1) einen Wäscher (2) mit kaltem oder gekühltem Wasser aufweist.

8. Vorrichtung nach Anspruch 7, **dadurch gekennzeichnet, dass** im Wasserkreislauf, vorzugsweise außerhalb des Wäschers (2), eine Kühlstufe (4) angeordnet ist.

9. Vorrichtung nach einem der Ansprüche 7 oder 8, **dadurch gekennzeichnet, dass** der Wäscher (2) eine Beregnungseinrichtung (5) umfasst.

10. Vorrichtung nach einem der Ansprüche 7 bis 9, **dadurch gekennzeichnet, dass** eine Frischwasserzufuhr (6) vorgesehen ist, über die Frischwasser dem Wäscher (2) zuführbar ist.

11. Vorrichtung nach einem der Ansprüche 1 bis 10, **dadurch gekennzeichnet, dass** das Gasgemisch vor Eintritt in die Wascheinrichtung (1) eine Temperatur zwischen 50° und 140°, vorzugsweise zwischen 60° und 100°, aufweist.

12. Vorrichtung nach einem der Ansprüche 1 bis 11, **dadurch gekennzeichnet, dass** das Gasgemisch vor Eintritt in die Wascheinrichtung (1) einen Überdruck zwischen 200 mbar und 2000 mbar, vorzugsweise zwischen 400 mbar und 1000 mbar, aufweist.

13. Vorrichtung nach einem der Ansprüche 1 bis 12, **dadurch gekennzeichnet, dass** das Gasgemisch nach Austritt aus der Wascheinrichtung (1) eine Temperatur zwischen 5° und 45°, vorzugsweise zwischen 15° und 25°, aufweist.

14. Vorrichtung nach einem der Ansprüche 1 bis 13, **dadurch gekennzeichnet, dass** durch die Entspannungseinrichtung (12) Druck und Temperatur des Gasgemisches reduzierbar sind.

15. Vorrichtung nach einem der Ansprüche 1 bis 14, **dadurch gekennzeichnet, dass** die Entspannungseinrichtung (12) mindestens eine Entspannungsmaschine, vorzugsweise eine Turbine, aufweist.

16. Vorrichtung nach Anspruch 15, **dadurch gekennzeichnet, dass** die Entspannungsmaschine über eine Welle mit einem Laufrad des Verdichters (9) verbindbar ist.

17. Vorrichtung nach einem der Ansprüche 1 bis 16, **dadurch gekennzeichnet, dass** das Gasgemisch nach dem Austritt aus der Entspannungseinrichtung (12) eine Temperatur zwischen 5° und 45°, vorzugsweise zwischen 5° und 60°, aufweist.

18. Vorrichtung nach einem der Ansprüche 1 bis 17, **dadurch gekennzeichnet, dass** das Gasgemisch nach dem Austritt aus der Entspannungseinrichtung (12) einen Überdruck zwischen 20 mbar und 300 mbar, vorzugsweise zwischen 80 mbar und 150 mbar, aufweist.

19. Vorrichtung nach einem der Ansprüche 1 bis 18, **dadurch gekennzeichnet, dass** das Gasgemisch nach dem Austritt aus der Entspannungseinrichtung (12) eine relative Feuchte zwischen 30% und 90%, vorzugsweise zwischen 40% und 80%, aufweist.

20. Vorrichtung nach einem der Ansprüche 1 bis 19, **dadurch gekennzeichnet, dass** das Gasgemisch nach der Konditionierung einer angeschlossenen Verbrennungsmaschine (13), vorzugsweise einem Biogasmotor, zuführbar ist.

21. Vorrichtung nach Anspruch 20, **dadurch gekennzeichnet, dass** das Gasgemisch vor Eintritt in die Verbrennungsmaschine (13) eine Temperatur zwischen 15° und 60°, vorzugsweise zwischen 25° und 40°, aufweist.

22. Vorrichtung nach Anspruch 20 oder 21, **dadurch gekennzeichnet, dass** das Gasgemisch vor Eintritt in die Verbrennungsmaschine (13) einen Überdruck zwischen 20 mbar und 300 mbar, vorzugsweise zwischen 80 mbar und 150 mbar, aufweist.

23. Vorrichtung nach einem der Ansprüche 20 bis 22, **dadurch gekennzeichnet, dass** das Gasgemisch vor Eintritt in die Verbrennungsmaschine (13) eine relative Feuchte zwischen 30% und 90%, vorzugsweise zwischen 50% und 80%, aufweist.

24. Verfahren zur Konditionierung eines Gasgemisches, insbesondere mittels einer Vorrichtung nach einem der Ansprüche 1 bis 23, , **dadurch gekennzeichnet, dass** das Gasgemisch zuerst verdichtet, danach mit kaltem und/oder mit gekühltem Wasser in Kontakt gebracht, vorzugsweise gewaschen, und danach wieder entspannt wird.

25. Verfahren nach Anspruch 24, **dadurch gekennzeichnet, dass** Wasser verwendet wird, welches kälter als das Gasgemisch ist.

26. Verfahren nach Anspruch 24 oder 25, **dadurch gekennzeichnet, dass** das Gasgemisch nach dem Entspannen, vorzugsweise mittels Wärmetauscher, erwärmt wird.
